# EUROPEAN PATENT SPECIFICATION

(11) **EP 2 054 037 B1**
(45) Date of publication and mention of the grant of the patent: **16.12.2009**
(21) Application number: 07788137.3
(22) Date of filing: 01.08.2007
(51) Int. Cl.: A61K 9/16

(54) **SUBCUTANEOUS IMPLANTS RELEASING AN ACTIVE PRINCIPLE OVER AN EXTENDED PERIOD OF TIME**
SUBKUTANE IMPLANTATE, DIE ÜBER EINEN LÄNGEREN ZEITRAUM EINEN WIRKSTOFF FREISETZEN
IMPLANTS SOUS-CUTANÉS LIBÉRANT UN PRINCIPE ACTIF PENDANT UNE DURÉE ÉTENDUE

(30) Priority: 02.08.2006 IT MI20061545
(43) Date of publication of application: 06.05.2009
(73) Proprietor: Mediolanum Pharmaceuticals Limited, Dublin 4 (IE)
(72) Inventor: MAURIAC, Patrice, 75012 Paris (FR); MARION, Pierre, 93360 Neuilly Plaisance (FR)
(74) Representative: Cattaneo, Elisabetta
(86) International application number: PCT/EP2007/057969
(87) International publication number: WO 2008/015238

(56) References cited:
- WO-A-00/33809
- US-B1- 6 183 781

## Description

### Field of the invention

The present invention relates to:
➢ Implants for subcutaneous administration obtained by extrusion, containing microparticles comprising an active ingredient dispersed in PLGA and microparticles consisting of the same or an other active ingredient preferably of the same therapeutic category as that contained in PLGA microparticles, all said particles being dispersed in a PLGA matrix, having a glass transition temperature lower than that of the PLGA contained in the aforesaid microparticles;
➢ the relative process for preparing said implants.

### State of the art

The advantage of using implants containing controlled release drugs is well known in the state of the art. Many active ingredients are rapidly metabolized and eliminated by the human or mammalian organism, therefore requiring frequent administration of the drug with the aim of maintaining an adequate therapeutic concentration.

An example of controlled release implants are represented by subcutaneous implants.

Among the numerous implants already known, the subcutaneous implants described in WO00/33809 represent a net improvement if compared to the previous subcutaneous implants containing as active principle a polypeptide dispersed in a matrix of polylactic-glycolic acid in that they are able to release the aforesaid active principle in 6 months. The subcutaneous implants described in said previous patent differ also in that they present an essentially triphasic and not biphasic release profile namely: release by pure diffusion, release by swelling controlled diffusion and release by polymer degradation.

This progression therefore allows for an extension of release times. In fact when these implants are introduced into an aqueous medium, water diffuses through the polymeric matrix reaching the peptide particles closest to the surface and subsequently the inner zones of the implant.

The implant remains substantially unmodified for about 6 weeks and in this period releases approximately 30% of the peptide.

The duration of this stage of pure diffusion is essentially determined by the level of heterogeneity of the peptide dimensions and the rate is essentially determined by the particle content in the PLGA matrix.

As the active principle presents a diversity of dimensions, a sufficient quantity of peptide remains after the first stage of dissolution and can be released in the successive stages mentioned, that is release by diffusion and swelling, or release by disintegration of the polymer.

These implants although representing a considerable advantage, they suffer from the following drawback.

Being characterised by having a three-release profile, it happens that after the first drug release phase and before the second release phase starts, a lag time is observed lasting in some cases many days, in which the drug is poorly or not released.

In some case and for some therapeutic protocols this interruption in the drug release should be avoided and a more linear release profile might be rendered necessary.

The need was therefore felt to overcome this drawback.

### Summary of the invention

Now the Applicant has found subcutaneous implants that solve the aforementioned problems

These implants for subcutaneous administration, which are obtained by extrusion, contain microparticles comprising an active ingredients dispersed in PLGA and microparticles consisting of the same active ingredient, all said microparticles being dispersed in a PLGA matrix, having a glass transition temperature lower than that of the PLGA contained in the aforesaid microparticles.

A further subject of the present invention relates to subcutaneous implants containing the aforementioned PLGA microparticles in which is dispersed the active principle and optionally microparticles consisting of a different active ingredient of the same therapeutic category as that contained in the PLGA particles.

In fact the applicant has found that with these subcutaneous implants further subject of to the present invention, the drug released from PLGA microparticles is very similar to that of conventional subcutaneous implant containing twice the weight of the same active ingredient.

In addition with the subcutaneous implants of the invention it is possible to protect the amount of active agent to be released at the end of the dissolution process. During the initial phase of the release, mainly the active ingredient is released which is dispersed in PLGA forming the implant matrix, whereas the active ingredient contained in the PLGA particles is more protected from water and acidity of the outer PLGA and it is released later.

### Description of the figures

Figure 1 represents a schematic section view along the axis I-I of the subcutaneous implants according to the present invention.
Figure 2 shows the active ingredient release profile (%of the total amount released in ordinate versus time in abscissae of the subcutaneous implants 2#1 and 2#2 prepared as described in Example 2.
Figure 3 shows the total peptide release profile (%of the amount released in ordinate versus time in abscissae of the subcutaneous implants), the Avorelin release and leuprorelin release from the subcutaneous implant 2#3 prepared as described in Example 2.
Fig. 4 shows the Avorelin release(%of the total amount released in ordinate versus time in abscissae of the subcutaneous implants from the conventional subcutaneous implant 2#1 in comparison with Avorelin release from the subcutaneous implant 2#3 of the invention as prepared in Example 2.
Figure 5 shows the Medroxy Progesterone Acetate (MPA) release (%of the amount released in ordinate versus time in abscissae of the subcutaneous implants) from the conventional subcutaneous implant 3#1 and the subcutaneous implant of the invention 3#3.
Figure 6 shows the Medroxy Progesterone Acetate (MPA) release profile (%of the total amount released in ordinate versus time in abscissae of the subcutaneous implants) from the conventional subcutaneous implant 3#2 and the subcutaneous implant of the invention 3#4.
Fig. 7 shows the Fentanyl Citrate release profile (%of the amount of drug released in ordinate versus time in abscissae ) from the conventional implants 4#1 and the subcutaneous implants 4#2.
Fig. 8 shows the fentanyl citrate release(%of the amount of drug released in ordinate versus time in abscissae) from the sole implant of the invention 4#2.

### Detailed description of the invention

Preferably in the subcutaneous implants of the invention the microparticles containing the active ingredient dispersed in PLGA are obtained with a process comprising the following steps:
a) dry mixing PLGA with the active ingredient, or (a') wet granulating the mixture of PLGA and active ingredient,
b) optionally drying the wet granulated mixture obtained in step (a') to obtain a residue containing a minimum liquid content of between 0.1 and 3%,
c) extruding the mixture coming from step (b) or the dry mixture coming from step (a).
d) grinding and sieving the extruded product coming from step (c) thereby obtaining microparticles having a size as determined by sieving lower than 500µm, more preferably in the fraction [50;250 µm].

The solvent optionally used in step (a') is for example ethanol or water.

The subcutaneous implants of the present invention preferably contain an active ingredient chosen from the group consisting of: a peptide, an active principle able to increase bone density, an analgesic-narcotic active principle, a steroid hormone, for hormone treatments during menopause and for contraception.

More preferably said peptide is chosen from: avorelin, triptorelin, goserelin and leuprorelin.

The active ingredient able to increase bone density are preferably chosen from: pharmaceutically acceptable bisphosphonic acids and their salts, vitamin D or analogues thereof and sex hormones.

Of these bisphosphonic acids and their pharmaceutically acceptable related salts, we mention for example the compounds of general formula (I): 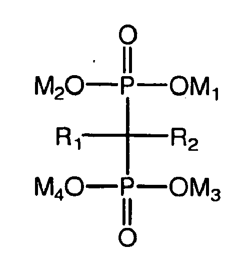 in which M₁, M₂, M₃ and M₄ are monovalent cations and/or H, where said monovalent cations are chosen from alkaline metals, or cations of aliphatic or cycloaliphatic amines, and even more preferably said cations are Na⁺, we cite for example those in which R₁ and R₂ have the meanings given in the following table 1:

**Table 1**

| | | |
|---|---|---|
| Bisphosphonate | R₁ | R₂ |
| Etidronate | OH | CH₃ |
| Chlodronate | Cl | Cl |
| Pamidronate | OH | CH₂CH₂NH₂ |
| Alendronate | OH | CH₂ CH₂ CH₂NH₂ |
| Risedronate | OH | CH₂-3-pyridine |
| Tiludronate | H | CH₂-S-phenyl-4Cl |
| Ibandronate | OH | CH₂CH₂N(CH₃)pentyl |
| Zoledronate | OH | CH₂CH₂-1-imidazole |
| Minodronate | OH | CH₂CH₂-2-imidazopyridinyl |
| Incadronate | OH | N-(cycloheptyl) |
| Olpadronate | OH | CH₂CH₂N(CH₃)₂ |
| Neridronate | OH | CH₂CH₂CH₂CH₂CH₂NH₂ |
| EB1053 | OH | CH₂-1-pyrrolidinyl |

Particularly preferred are etidronate disodium, alendronate disodium and pamidronate disodium.

Preferably the sex hormones are selected from the group of estrogens and progestins and of the latter, androgenic progestins are more preferably used. Estrogens are of steroid type and are chosen from the class consisting of estradiol, estradiol valerate, estradiol cypionate, estrone, estrone sulphate or estrogens of non-steroidal type for example diethylstilbestrol, p-p'-DDT, bisphenyl-A.

Among male progestins preferred are those chosen from the class consisting of norethindrone, norethinodrel, norgestrel, desogestrel, norgestimate.

As "drugs with narcotic analgesic activity" preferred are morphine and morphinans, i.e. compounds having a chemical structure and activity similar to that of morphine and µ receptor agonists, but also compounds with morphinic-type activity, in other words also µ receptor agonists but with a different chemical structure such as those belonging to the phenylpiperidine class. (Goodman & Gilman's "The pharmacological basis of therapeutics "Ninth Edition Chapter 23 pages 521-555). As phenylpiperidine µ receptor agonists we cite as preferred at least one active principle chosen from the class consisting of meperidine, fentanyl and relative pharmaceutically acceptable salts, fentanyl congeners, for example sufentanyl, alfentanyl, lofentanyl, carfentanyl, remifentanyl and their pharmaceutically acceptable salts

The active principle present in the particles of the invention can present heterogeneous dimensions or can have a more homogeneous particle size distribution.

Preferably, in the subcutaneous implants of invention the active ingredient contained in PLGA particles and the active ingredient dispersed in the PLGA forming the matrix of the implant, shows a heterogeneous size distribution ranging from 1 to 63µm or from 1 to 100 µm.

The PLGA contained in the microparticles in the subcutaneous implants of the invention has preferably an average molecular weight ranging from 50000 Da to 150000 Da and a molar ratio of lactic acid/ glycolic acid ranging from 50/50 to 75/25.

The PLGA forming the matrix of the subcutaneous implants according to the present invention has preferably an average molecular weight ranging from 10000 Da to 40000 Da and a molar ratio of lactic acid/ glycolic acid ranging from 50/50 to 60/40.

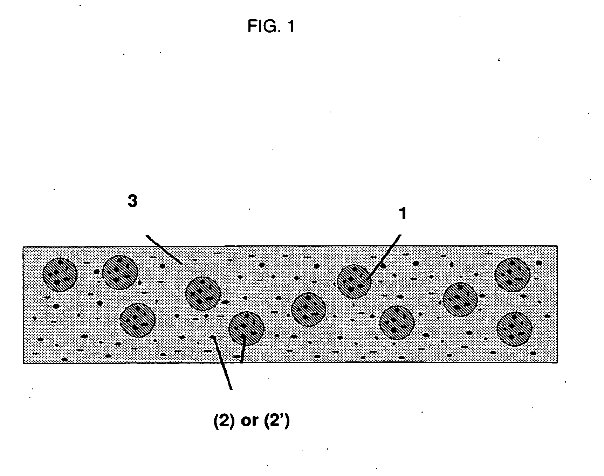

Figure 1 reports a schematic view section of a preferred embodiment of the subcutaneous implant according to the present invention containing besides PLGA particles (1) in which is dispersed an active ingredient according to the present also the particles (2) of the same active ingredient or of a different active ingredient (2') from that contained in (1) In this figure (3) indicates a PLGA forming the scaffold of the implant and having a glass transition temperature lower than that used for preparing (1).

The present invention further relates to the preparation of the subcutaneous implants according to the present invention with a process comprising the following steps:
(A) dry mixing the following components (1) the microparticles according to the present invention and (2) the particles consisting only of the same active ingredient contained in (1) or (2') particles consisting of a different active ingredient, preferably being in the same therapeutic category as that contained in (1), and (3) PLGA having a glass transition temperature lower than that of PLGA utilised for preparing (1) or (A') wet granulating in a suitable solvent such as water or a lower alcohol, preferably ethanol, a mixture consisting of the components (1), (3), and optionally (2) or (2'),
(B) drying the wet granulated mixture coming from step (A'),
(C) extruding the mixture coming from step (A) or from step (B).

We report herewith the following examples of preparation of the particles according to the present invention.

### EXAMPLE 1- preparation of the microparticels containing Avorelin and PLGA.

Subcutaneous implants containing 25% w/w Avorelin (having particle size distribution ranging from 1 to 63 µm) and PLGA (UG molar ratio 54/46 - average molecular weight 51,000 Da) are prepared as described in WO00/33809 and ground using a rotating mill. This is followed by sieving the microparticles to select those comprised within the fraction [50µm;250µm].

### EXAMPLE 2-subcutaneous implants containing peptides

**Formulation 2#1:** Subcutaneous implants containing 25% w/w Avorelin (having particle size distribution ranging from 1 to 63 µm) and PLGA (UG molar ratio 50/50 - average molecular weight 100,000 Da) are prepared as described in WO00/33809.

**Formulation 2#2:** Subcutaneous implants containing 39% w/w Avorelin (having particle size distribution ranging from 1 to 63 µm) and PLGA (UG molar ratio 50/50 - average molecular weight 100,000 Da) are prepared as described in WO00/33809 and ground using a rotating knife grinder as described in Example 1. This is followed by sieving the particles in order to select particles comprised within the [ 50 ; 250 µm ] interval

Subcutaneous implants containing 25% m/m Avorelin are prepared by mixing and extruding 13% m/m of Avorelin (having particle size distribution ranging from 1 to 63 µm), 57% m/m of PLGA (UG molar ratio 50/50 - average molecular weight 17,000 Da) and 30% m/m of particles obtained as previously described. In those implants (containing 25% m/m of Avorelin), 12% m/m come from the particles and 13% m/m from the external matrix.

### Formulation 2#3:

Subcutaneous implants containing 26% w/w of LHRH agonist are prepared by mixing and extruding 14% w/w of Leuprorelin (having particle size distribution ranging from 1 to 63 µm), 56% w/w of PLGA (UG molar ratio 50/50 - average molecular weight 13,000 Da) and 30% w/w of particles obtained as previously described. In those implants (containing 26% w/w of LHRH agonists), 12% w/w (Avorelin) come from the particles and 14% w/w (Leuprorelin) from the external matrix.

The amount of Avorelin is identical in this formulation and in the particles included in the Formulation 2#2 implants. In this case, the peptide contained in the external matrix is very similar (leuprorelin) but not identical. Then, the amounts of Leuprorelin and Avorelin dissolved can be estimated separately at each sampling time point during the in vitro dissolution test. This allows knowing the release profile of avorelin from the particles without any analytical interference from the peptide released form the external matrix.

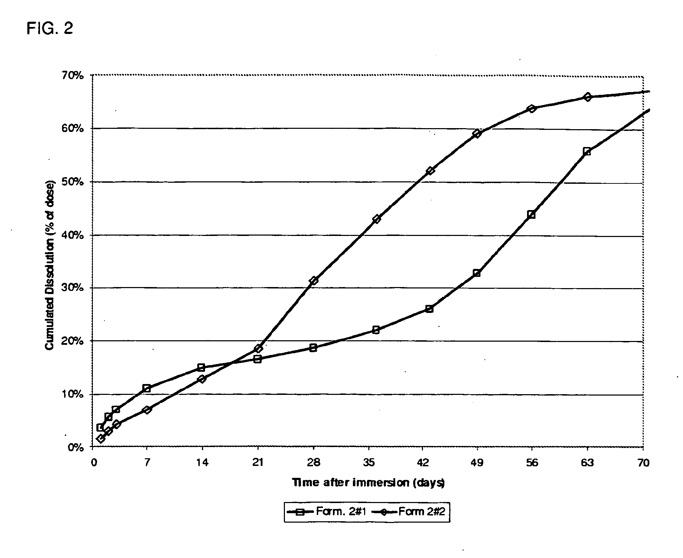

Figure 2 shows the active ingredient release profiles (% of the dose released versus time after immersion) from formulations 2#1 and 2#2.

The release profile obtained with formulation 2#2 is closer to the linearity than the one obtained with formulation 2#1

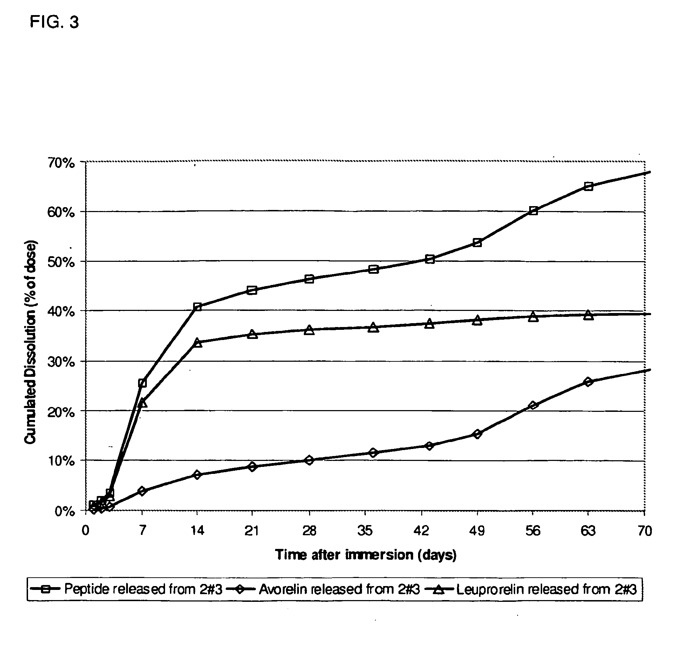

Figure 3 shows the active ingredients release profiles (% of the total amount released versus time after immersion) from the subcutaneous implant 2#3.

In this case, the way each peptide is released from either the particles or the matrix is elucidated.

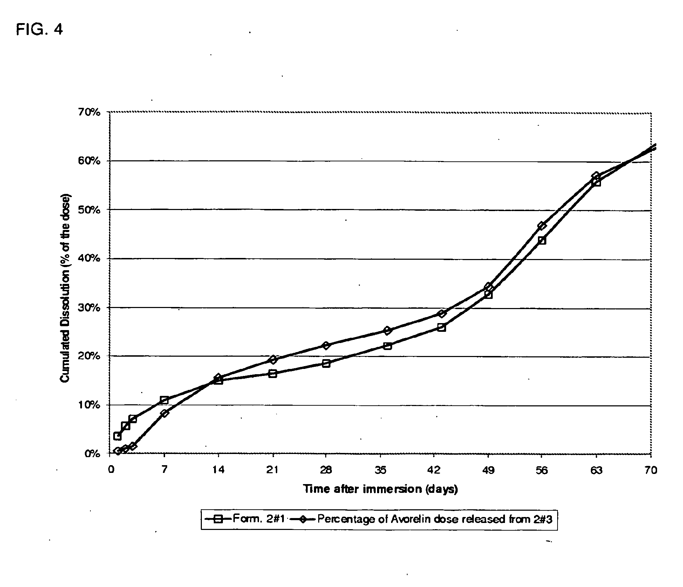

Figure 4 shows the active ingredient release profiles (% of the total amount released versus time after immersion) from formulation 2#1 and the avorelin release profile (% of the avorelin dose released versus time after immersion) from the subcutaneous implants 2#3.

A very similar Avorelin release profile (% of the dose released versus time) is observed between the particles contained in formulation 2#3 and the implants Nr 2#1, although the in the subcutaneous implants of the invention the content of this active ingredient is a half as that contained in 2#1.

### EXAMPLE 3- preparation of the subcutaneous implant containing Medroxy Progesterone Acetate (MPA)

**Formulation 3#1:** Subcutaneous implants containing 30% w/w MPA (having particle size distribution ranging from 1 to 63 µm) and PLGA (UG molar ratio 75/25 - average molecular weight 120,000 Da) are prepared as described in WO00/33809

**Formulation 3#2:** Subcutaneous implants containing 40% w/w MPA (having particle size distribution ranging from 1 to 63 µm) and PLGA (UG molar ratio 75/25 - average molecular weight 120,000 Da) are prepared as described in WO00/33809

**Formulation 3#3:** Subcutaneous implants containing 55% w/w MPA (having particle size distribution ranging from 1 to 63 µm) and PLGA (UG molar ratio 75/25 - average molecular weight 120,000 Da) are prepared as described in WO00/33809 (extrusion Temperature 120°C) and ground using a rotating knife grinder. This is followed by sieving the particles in order to select particles comprised within the [ 50 ; 250 µm ] interval

Subcutaneous implants containing 30% w/w MPA are prepared by mixing and extruding (Extrusion temperature 95°C) 21% w/w of MPA (having particle size distribution ranging from 1 to 63 µm), 62% w/w of PLGA (UG molar ratio 60/40 - average molecular weight 53,000 Da) and 17% w/w of particles obtained as previously described. In those implants (containing 30% w/w of MPA), 9% w/w come from the particles and 21 % w/w from the external matrix.

**Formulation 3#4:** Subcutaneous implants containing 25% w/w MPA (having particle size distribution ranging from 1 to 63 µm) and PLGA (UG molar ratio 75/25 - average molecular weight 120,000 Da) are prepared as described in WO00/33809 (extrusion Temperature 120°C) and ground using a rotating knife grinder. This is followed by sieving the particles in order to select particles comprised within the [ 50 ; 250 µm ] interval.

Subcutaneous implants containing 40% w/w MPA are prepared by mixing and extruding (Extrusion temperature 95°C) 13% w/w of MPA (having particle size distribution ranging from 1 to 63 µm), 37% w/w of PLGA (L/G molar ratio 60/40 - average molecular weight 53,000 Da) and 50% w/w of particles obtained as previously described. In those implants (containing 40% w/w of MPA), 27% w/w come from the particles and 13% w/w from the external matrix.

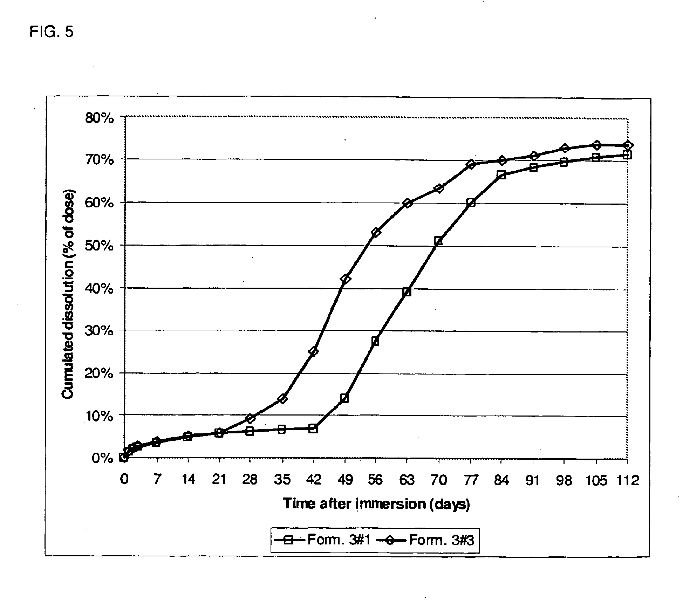

Figure 5 presents a comparison of the active ingredient release profiles (% of the total amount released versus time after immersion) from formulations 3#1 and 3#3 (30% w/w MPA loaded depots).

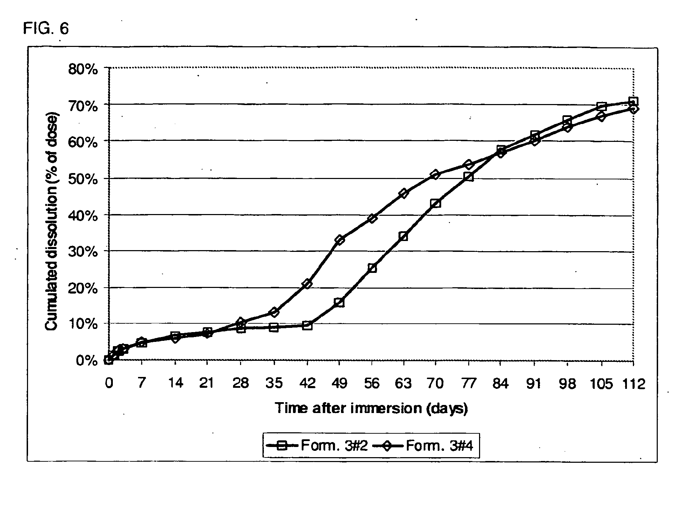

Figure 6 presents a comparison of the active ingredient release profiles (% of the total amount released versus time after immersion) from formulations 3#2 and 3#4 (40% w/w MPA loaded depots).

In both cases the "particles loaded depots" provide for a more linear release profile. This was mainly obtained by shortening the period of poor dissolution observed from day 14 to 42 with standard depots.

### EXAMPLE 4- preparation of the subcutaneous implant containing Fentanyl citrate

**Formulation 4#1:** Subcutaneous implants containing 42% w/w Fentanyl citrate (having particle size distribution ranging from 1 to 63 µm) and PLGA (UG molar ratio 75/25 - average molecular weight 120,000 Da) are prepared as described in WO00/33809

**Formulation 4#2:** Subcutaneous implants containing 36% w/w Fentanyl citrate (having particle size distribution ranging from 1 to 63 µm) and PLGA (UG molar ratio 75/25 - average molecular weight 120,000 Da) are prepared as described in WO00/33809 (extrusion Temperature 120°C) and ground using a rotating knife grinder. This is followed by sieving the particles in order to select particles comprised within the [ 50 ; 250 µm ] interval

Subcutaneous implants containing 42% w/w Fentanyl citrate are prepared by mixing and extruding (Extrusion temperature 95°C) 22% w/w of Fentanyl citrate (having particle size distribution ranging from 1 to 63 µm), 22% w/w of PLGA (L/G molar ratio 55/45 - average molecular weight 51,000 Da) and 56% w/w of particles obtained as previously described. In those implants (containing 42% w/w of Fentanyl citrate), 20% w/w come from the particles and 22% w/w from the external matrix.

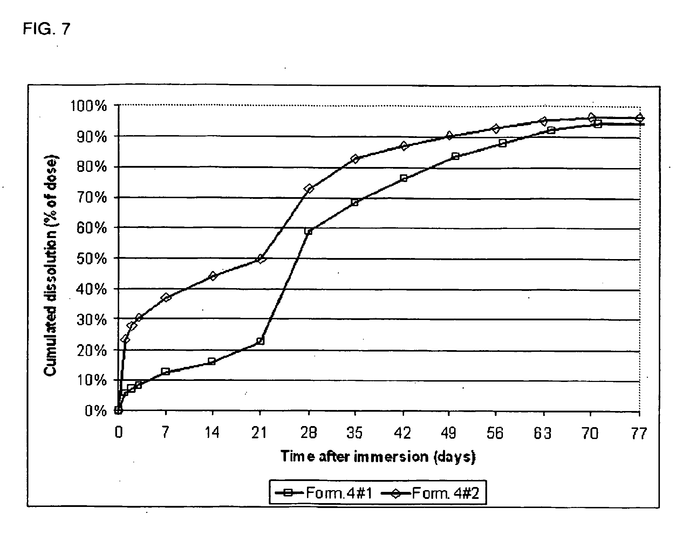

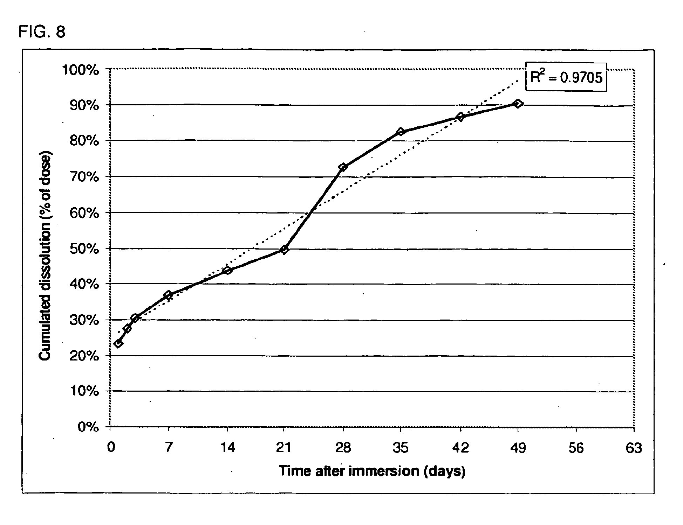

Figure 7 presents a comparison of the active ingredient release profiles (% of the total amount released versus time after immersion) from formulations 4#1 and 4#2. Here again "Particles loaded depots" provide for a more linear release profile. In this case, formulation 4#2 actually leads to a fairly linear release profile (R² = 0.9705 for % released = f(t) from week 1 to 7 calculated by linear regression - see Figure 8).

## Claims

1. Subcutaneous implants obtained by extrusion containing (1) microparticles containing an active ingredient dispersed in a PLGA matrix, all said microparticles (1) being dispersed in a matrix of PLGA (3), having a glass transition temperature lower than that of the PLGA contained in (1).

2. Subcutaneous implants according to claim 1, wherein the microparticles (1) are obtained by grinding or spheronising an extruded PLGA containing dispersed therein this active ingredient.

3. The subcutaneous implants according to claim 2 wherein said microparticles (1) are prepared with a process comprising the following steps:
a) dry mixing PLGA with the active ingredient, or optionally (a') wet granulating mixture of PLGA and active ingredient,
b) drying the wet granulated mixture obtained in step (a') to obtain a residue containing a minimum liquid content of between 0.1 and 3%,
c) extruding the mixture coming from step (b) or the dry mixture coming from step (a).
d) grinding and sieving the extruded product coming from step (c) thereby obtaining microparticles having a size as determined by sieving lower than 500µm.

4. The subcutaneous implants according to claim 3, wherein in step (d) the extruded product is ground until obtaining microparticles having a size as determined by sieving in the fraction [50;250 µm].

5. The subcutaneous implants according to anyone of claims 1-4, containing besides the microparticles (1) also the microparticles (2) consisting of the same active ingredient contained in (1) or the microparticles (2') consisting of a different active ingredient.

6. The subcutaneous implants according to claim 5, wherein microparticles (2') are of an active ingredient of the same therapeutic category as that of the active ingredient contained in (1).

7. The subcutaneous implants according to claim 5 or 6, wherein the active ingredient contained in (1) and the microparticles (2) or (2') are selected from the group consisting of a peptide, an active ingredient able to increase bone density, an analgesic-narcotic active principle, a steroid hormone for hormone treatments during menopause and for contraception.

8. The subcutaneous implants according to claim 7 wherein the peptide is selected from the group consisting of avorelin, triptorelin, goserelin and leuprorelin.

9. The subcutaneous implants according to claim 7 wherein the active ingredient able to increase bone density is chosen from: pharmaceutically acceptable bisphosphonic acids and pharmaceutically acceptable salts thereof, vitamin D or analogues thereof and sex hormones.

10. The subcutaneous implants according to claim 9, wherein these biphosponic acid salts are selected from etidronate disodium, alendronate disodium and pamidronate disodium.

11. The subcutaneous implants according to claim 9 wherein said sex hormones are selected from the group of estrogens and androgenic progestins.

12. The subcutaneous implants according to claim 11, wherein said estrogens are chosen from the class consisting of estradiol, estradiol valerate, estradiol cypionate, estrone, estrone sulphate or estrogens of non-steroidal type.

13. The subcutaneous implants according to claim 11 wherein said androgenic progestins are chosen from the class consisting of norethindrone, norethinodrel, norgestrel, desogestrel, norgestimate.

14. The subcutaneous implants according to claim 7 wherein the active ingredient with narcotic analgesic activity is selected from the group consisting of morphine and morphinans, and µ receptor agonists.

15. The subcutaneous implants according to claim 14, wherein said µ receptor agonists are phenylpiperidines selected from the group consisting of: meperidine, fentanyl and related pharmaceutically acceptable salts, fentanyl congeners.

16. The subcutaneous implants according to anyone of claim 11-15, wherein the active ingredient contained in (1) or the microparticles (2) o (2') has homogeneous or heterogeneous particles size distribution.

17. The subcutaneous implants according to claim 16, wherein the active ingredient has a heterogeneous particles size distribution.

18. The subcutaneous implants according to claim 17 wherein the heterogeneous particles size distribution range from 1 to 63µm or from 1 to 100 µm.

19. The subcutaneous implants according to anyone of claims 1-18, wherein the PLGA contained in (1) has an average molecular weight ranging from 50000 Da to 150000 Da and a molar ratio of lactic acid/ glycolic acid ranging from 50/50 to 75/25.

20. The subcutaneous implants according to anyone of claims 1-19, wherein the PLGA (3) has an average molecular weight ranging from 10000 Da to 40000 Da and a molar ratio of lactic acid/ glycolic acid ranging from 50/50 to 60/40.

21. A process for preparing the subcutaneous implant according anyone of claims 1-19, comprising the following steps:
A) dry mixing particles (1) with PLGA (3) and optionally the active ingredient (2) or (2') or (A') wet granulating particles (1) with PLGA (3) and optionally with (2) or (2')
(B) drying the wet granulated mixture coming from step (A'),
(C) extruding the mixture coming from step (A) or from step (B).

## Patentansprüche

1. Subkutane Implantate, erhalten durch Extrusion, enthaltend (1) Mikroteilchen, enthaltend einen aktiven Wirkstoff, der in einer PLGA-Matrix dispergiert ist, wobei alle genannten Mikroteilchen (1) in einer Matrix aus PLGA (3) dispergiert sind, das eine Glasübergangstemperatur hat, die niedriger ist als diejenige der PLGA, die in (1) enthalten ist.

2. Subkutane Implantate gemäß Anspruch 1, worin die Mikroteilchen (1) erhalten werden durch Mahlen oder Sphäronisieren einer extrudierten PLGA, die darin diesen aktiven Wirkstoff dispergiert enthält.

3. Subkutane Implantate gemäß Anspruch 2, worin die Mikroteilchen (1) hergestellt werden mit einem Verfahren, das die folgenden Schritte umfasst:
(a) Trocken-Mischen von PLGA mit dem aktiven Wirkstoff, oder gegebenenfalls (a') Nass-Granulieren einer Mischung von PLGA und des aktiven Wirkstoffs;
(b) Trocknen der nass-granulierten Mischung, die in Schritt (a') erhalten wurde, unter Erhalt eines Rückstandes, der einen minimalen Flüssigkeits-Gehalt von zwischen 0,1 und 3 % enthält;
(c) Extrudieren der aus Schritt (b) kommenden Mischung oder der aus Schritt (a) kommenden Trocken-Mischung;
(d) Mahlen und Sieben des aus Schritt (c) kommenden extrudierten Produktes, wodurch man Mikroteilchen mit einer Größe, bestimmt durch Sieben, von weniger als 500 µm erhält.

4. Subkutane Implantate gemäß Anspruch 3, worin in Schritt (d) das extrudierte Produkt bis zum Erhalt von Mikroteilchen gemahlen wird, die eine Größe, bestimmt durch Sieben, in der Fraktion [50;250µm] aufweisen

5. Subkutane Implantate gemäß irgendeinem der Ansprüche 1 bis 4, enthaltend neben den Mikroteilchen (1) auch die Mikroteilchen (2), die aus demselben aktiven Wirkstoff bestehen, enthalten in (1), oder die Mikroteilchen (2'), die aus einem unterschiedlichen aktiven Wirkstoff bestehen.

6. Subkutane Implantate nach Anspruch 5, worin Mikroteilchen (2') aus einem aktiven Wirkstoff derselben therapeutischen Kategorie wie derjenigen des aktiven Wirkstoffs bestehen, der in (1) enthalten ist.

7. Subkutane Implantate gemäß Anspruch 5 oder 6, worin die aktive Komponente, die in (1) und den Mikroteilchen (2) oder (2') enthalten ist, gewählt ist aus der Gruppe, die besteht aus einem Peptid, einem aktiven Wirkstoff, der in der Lage ist, die Knochendichte zu erhöhen, einem analgetisch-narkotischen aktiven Prinzip, einem Steroid-Hormon für Hormon-Behandlung während der MenoPause und als Verhütungsmittel.

8. Subkutane Implantate nach Anspruch 7, worin das Peptid gewählt ist aus der Gruppe, die besteht aus Avorelin, Triptorelin, Goserelin und Leuprorelin.

9. Subkutane Implantate nach Anspruch 7, worin der aktive Wirkstoff, der in der Lage ist, die Knochendichte zu erhöhen, gewählt ist aus pharmazeutisch annehmbaren Biphosphonsäuren und pharmazeutisch annehmbaren Salzen davon, Vitamin D oder Analogen davon und Sexual-Hormonen.

10. Subkutane Implantate nach Anspruch 9, worin diese Biphosphonsäure-Salze gewählt sind aus Etidronat-Dinatrium, Alendronat-Dinatrium und Pamidronat-Dinatrium.

11. Subkutane Implantate nach Anspruch 9, worin die Sexual-Hormone gewählt sind aus der Gruppe Östrogene und androgene Progestine.

12. Subkutane Implantate nach Anspruch 11, worin die Östrogene gewählt sind aus der Klasse, die besteht aus Estradiol. Estradiolvalerat, Estradiolcypionat, Estron, Estronsulfat oder Östrogenen des nicht-steroidalen Typs.

13. Subkutane Implantate nach Anspruch 11, worin die androgenen Progestine gewählt sind aus der Klasse, die besteht aus Norethindron, Norethinodrel, Norgestrel, Desogestrel, Norgestimat.

14. Subkutane Implantate nach Anspruch 7, worin der aktive Wirkstoff mit narkotisch-analgetischer Aktivität gewählt ist aus der Gruppe, die besteht aus Morphin und Morphinanen und µ-Rezeptor-Agonisten.

15. Subkutane Implantate nach Anspruch 14, worin die µ-Rezeptor-Agonisten Phenylpiperidine sind, die gewählt sind aus der Gruppe, die besteht aus Meperidin, Fentanyl und verwandten pharmazeutisch annehmbaren Salzen, Fentanyl-Artverwandten.

16. Subkutane Implantate nach irgendeinem der Ansprüche 11 bis 15, worin der aktive Wirkstoff, der in (1) oder den Mikroteilchen (2) oder (2') enthalten ist, eine homogene oder heterogene Teilchengröße-Verteilung hat.

17. Subkutane Implantate nach Anspruch 16, worin der aktive Wirkstoff eine heterogene Teilchengröße-Verteilung hat.

18. Subkutane Implantate nach Anspruch 17, worin die heterogene Teilchengröße-Verteilung im Bereich von 1 bis 63 µm oder im Bereich von 1 bis 100 µm liegt.

19. Subkutane Implantate gemäß irgendeinem der Ansprüche 1 bis 18, worin die in (1) enthaltene PLGA ein mittleres Molekular-Gewicht im Bereich von 50.000 Da bis 150.000 Da und ein Mol-Verhältnis Milchsäure/Glycolsäure aufweist, das im Bereich von 50/50 bis 75/25 liegt.

20. Subkutane Implantate gemäß irgendeinem der Ansprüche 1 bis 19, worin die PLGA (3) ein mittleres Molekulargewicht im Bereich von 10.000 Da bis 40.000 Da und ein Molverhältnis Milchsäure/Glycolsäure aufweist, das im Bereich von 50/50 bis 60/40 liegt.

21. Verfahren zur Herstellung von subkutanen Implantaten nach irgendeinem der Ansprüche 1 bis 19, umfassend die folgenden Schritte:
A) Trocken-Mischen von Teilchen (1) mit PLGA (3) und gegebenenfalls der aktiven Komponente (2) oder (2') oder (A') Nass-Granulieren von Teilchen (1) mit PLGA (3) und gegebenenfalls mit (2) oder (2');
B) Trocknen der nass-granulierten Mischung, die aus Schritt (A') kommt;
C) Extrudieren der aus Schritt (A) oder aus Schritt (B) kommenden Mischung.

## Revendications

1. Implants sous-cutanés obtenus par extrusion, contenant (1) des microparticules contenant un ingrédient actif dispersé dans une matrice de PLGA, toutes lesdites microparticules (1) étant dispersées dans une matrice de PLGA (3) ayant une température de transition vitreuse inférieure à celle du PLGA contenu dans (1).

2. Implants sous-cutanés selon la revendication 1, dans lesquels les microparticules (1) sont obtenues en broyant ou en sphéronisant un PLGA extrudé contenant cet ingrédient actif dispersé à l'intérieur.

3. Implants sous-cutanés selon la revendication 2, dans lesquels lesdites microparticules (1) sont préparées en utilisant un procédé comportant les étapes suivantes consistant à :
a) mélanger à sec du PLGA avec l'ingrédient actif, ou facultativement (a') granuler par voie humide un mélange de PLGA et d'ingrédient actif,
b) sécher le mélange granulé humide obtenu à l'étape (a') afin d'obtenir un résidu contenant une teneur minimale en liquide entre 0,1 et 3 %,
c) extruder le mélange provenant de l'étape (b) ou le mélange sec provenant de l'étape (a),
d) broyer et tamiser le produit extrudé provenant de l'étape (c) en obtenant ainsi des microparticules ayant une taille telle que déterminée par un tamisage inférieur à 500 µm.

4. Implants sous-cutanés selon la revendication 3, dans lesquels à l'étape (d), le produit extrudé est broyé jusqu'à l'obtention de microparticules ayant une taille telle que déterminée par tamisage dans la fraction [50;250 µm].

5. Implants sous-cutanés selon l'une quelconque des revendications 1 à 4, contenant également, en plus des microparticules (1), les microparticules (2) constituées du même ingrédient actif contenu dans (1) ou les microparticules (2) constituées d'un autre ingrédient actif.

6. Implants sous-cutanés selon la revendication 5, dans lesquels des microparticules (2') sont constituées d'un ingrédient actif de la même catégorie thérapeutique que celle de l'ingrédient actif contenu dans (1).

7. Implants sous-cutanés selon la revendication 5 ou 6, dans lesquels l'ingrédient actif contenu dans (1) et les microparticules (2) ou (2') sont choisis parmi le groupe constitué d'un peptide, d'un ingrédient actif capable d'accroître la densité osseuse, d'un principe actif analgésique-narcotique, d'une hormone stéroïde pour les traitements hormonaux pendant la ménopause et pour la contraception.

8. Implants sous-cutanés selon la revendication 7, dans lesquels le peptide est choisi parmi le groupe constitué d'avoréline, de triptoréline, de goséréline et de leuproréline.

9. Implants sous-cutanés selon la revendication 7, dans lesquels l'ingrédient actif capable d'accroître la densité osseuse est choisi parmi : les acides bisphosphoniques pharmaceutiquement acceptables et les sels pharmaceutiquement acceptables de ceux-ci, la vitamine D ou les analogues de celle-ci et les hormones sexuelles.

10. Implants sous-cutanés selon la revendication 9, dans lesquels ces sels d'acides bisphosphoniques sont choisis parmi l'étidronate disodique, l'alendronate disodique et le pamidronate disodique.

11. Implants sous-cutanés selon la revendication 9, dans lesquels lesdites hormones sexuelles sont choisies parmi le groupe constitué d'estrogènes et de progestines androgéniques.

12. Implants sous-cutanés selon la revendication 11, dans lesquels lesdits estrogènes sont choisis parmi la classe constituée d'estradiol, de valérate d'estradiol, de cypionate d'estradiol, d'estrone, de sulfate d'estrone ou d'estrogènes de type non-stréroïdien.

13. Implants sous-cutanés selon la revendication 11, dans lesquels lesdites progestines androgéniques sont choisies parmi la classe constituée de noréthindrone, de noréthinodrel, de norgestrel, de désogestrel et de norgestimate.

14. Implants sous-cutanés selon la revendication 7, dans lesquels l'ingrédient actif ayant une activité narcotique-analgésique est choisi parmi le groupe constitué de morphine et de morphinanes, et d'agonistes du récepteur µ.

15. Implants sous-cutanés selon la revendication 14, dans lesquels lesdits agonistes du récepteur µ sont des phénylpipéridines choisies parmi le groupe constitué du mépéridine, du fentanyl et sels pharmaceutiquement acceptables associés, des congénères du fentanyl.

16. Implants sous-cutanés selon l'une quelconque des revendications 11 à 15, dans lesquels l'ingrédient actif contenu dans (1) ou les microparticules (2) ou (2') a une distribution de taille de particules homogène ou hétérogène.

17. Implants sous-cutanés selon la revendication 16, dans lesquels l'ingrédient actif a une distribution de taille de particules hétérogène.

18. Implants sous-cutanés selon la revendication 17, dans lesquels la distribution de taille de particules hétérogène varie de 1 à 63 µm ou de 1 à 100 µm.

19. Implants sous-cutanés selon l'une quelconque des revendications 1 à 18, dans lesquels le PLGA contenu dans (1) a un poids moléculaire moyen variant de 50 000 Da à 150 000 Da et un rapport molaire acide lactique/acide glycolique variant de 50/50 à 75/25.

20. Implants sous-cutanés selon l'une quelconque des revendications 1 à 19, dans lesquels le PLGA (3) a un poids moléculaire moyen variant de 10 000 Da à 40 000 Da et un rapport molaire d'acide lactique/acide glycolique variant de 50/50 à 60/40.

21. Procédé pour préparer l'implant sous-cutané selon l'une quelconque des revendications 1 à 19, comportant les étapes suivantes consistant à :
(A) mélanger à sec des particules (1) avec du PLGA (3) et facultativement l'ingrédient actif (2) ou (2'), ou (A') granuler par voie humide des particules (1) avec du PLGA (3) et facultativement avec (2) ou (2'),
(B) sécher le mélange granulé humide provenant de l'étape (A'),
(C) extruder le mélange provenant de l'étape (A) ou de l'étape (B).
